# EUROPEAN PATENT APPLICATION

(11) **EP 2 579 044 A1**
(43) Date of publication of application: **10.04.2013**
(21) Application number: 11789779.3
(22) Date of filing: 31.05.2011
(51) Int. Cl.: G01N 35/00, G01N 35/08, G01N 35/10

(54) **ANALYSIS DEVICE, ANALYSIS SYSTEM AND ANALYSIS METHOD**

(30) Priority: 31.05.2010 JP 2010125194
(71) Applicant: Arkray, Inc., Kyoto-shi, Kyoto 601-8045 (JP)
(72) Inventor: FUJIMOTO, Koji, Kyoto-shi Kyoto 602-0008 (JP); FURUSATO, Noriaki, Kyoto-shi Kyoto 602-0008 (JP)
(74) Representative: Piésold, Alexander James
(86) International application number: PCT/JP2011/062437
(87) International publication number: WO 2011/152376

(57) **Abstract**

An analysis device of performing predetermined analysis processing to a sample. The analysis device includes: a first analysis unit A1, a second analysis unit A2 and a simple analysis unit A3 to perform a first analysis process to the sample, a second analysis process to the sample and a short-time analysis process to the sample, by which an analysis result is given in a shorter time than by the first analysis process, respectively; and a judging unit 63 to judge, before a result of the first analysis process is given, whether or not to perform the second analysis process on a basis of a result of the first analysis process.

## Description

### Technical Field

The present invention relates to an analysis device, an analysis system, and an analysis method to perform predetermined analysis processing to a sample.

### Background Art

Over the recent years, as analysis device or analysis system to analyze a sample (an analyte) such as blood and urine, the analysis device or analysis system which performs a plurality of analysis processes to the same sample has been developed. For example, in the patent documents 1-4, systems each including several analysis device which performs different analysis processes and a carry system which carries a plurality of sample containers sequentially to these analysis devices, are described. In such a system, at each analysis device, sample to be analyzed is obtained from the sample container carried by the carry system using a nozzle to obtain sample. Then, each analysis process is performed to the obtained sample.

Here, in the analysis device or analysis system which performs two or more analysis processes, there are cases where an analysis process to a sample is performed first, and then, after judging, on the basis of the result of the analysis process, whether or not to perform another analysis process to the sample, the another process to the sample is performed. However, with such a procedure, an analysis process to a sample and another analysis process to the sample cannot be performed simultaneously. There is therefore a possibility that the overall analysis performance degrades.

Moreover, also in cases where the progress state of an analysis process is affected by the progress status of another analysis process, there is a possibility that the overall analysis performance degrades.

### Prior Art Documents

Patent Document 1: Japanese Patent No. 3616744
Patent Document 2: Japanese Laid-open Patent Publication No. 07-92171
Patent Document 3: Japanese Patent No. 3031242
Patent Document 4: Japanese Patent No. 3031374

### Summary of Invention

It is an object of the present invention, which was devised under such circumstances, to provide technology of performing two or more analysis processes to a sample, which is capable of improving performance of the overall analysis processing.

In the present invention, to accomplish the above object, the following configurations are adopted.

An analysis device according to first invention includes: a short-time analysis unit to perform a short-time analysis process to a sample, which is completed in a shorter time than a first analysis process to the sample; and a judging unit to judge, on a basis of a result of the short-time analysis process, whether or not to perform a second analysis process different from the first short-time analysis process.

Namely, the analysis device according to the first invention has a function of judging (determining), on the basis of the result of the short-time analysis process (the analysis process that is completed in a shorter time than a first analysis process to the sample, such as process to measure a specific physical property of the sample) to a sample, whether or not to perform the second analysis process to the sample. With this analysis device used, the second analysis process to a sample can be started before the first analysis process to the sample is completed, Consequently, according to this analysis device, it is possible to improve overall performance in analyzing the sample using analysis processes.

The analysis device according to the first invention may include "a second analysis unit to perform the second analysis process to the sample" and/or "a first analysis unit to perform the first analysis process to the sample." Further, when carrying out the analysis device according to the first invention as the device having the second analysis unit, it is possible to adopt the judging unit, when judging that the second analysis process to the sample is to be performed, makes the second analysis unit start the second analysis process to the sample without waiting for completion of the first analysis process.

An analysis device according to a second invention includes : a short-time analysis unit to measure a specific physical property of a sample; a judging unit to judge, on a basis of a measurement result of the specific physical property by the short-time analysis unit, whether or not to perform a sediment test to the sample; and a sediment testing unit to perform the sediment test to the sample, the sediment test to which is judged to be performed by the judging unit.

That is, this analysis device has such a configuration that judges whether or not to perform the sediment test by measuring the specific physical property (specific gravity, value representing color tone) of urine sample, and perform the sediment test to the urine sample for which the sediment test is needed. Consequently, with this analysis device, it is possible to perform the urine test in a way that the sediment test (sediment analysis process) is started before the qualitative test (qualitative analysis process) is completed (in other words, in a may that overall performance in analyzing the urine sample is improved).

An analysis device according to a third invention is device of performing predetermined analysis processing to a sample, and includes: a first analysis unit, a second analysis unit and a short-time analysis unit to perform a first analysis process to the sample, a second analysis process to the sample and a short-time analysis process to the sample, by which an analysis result is given in a shorter time than by the first analysis process, respectively; and a judging unit to judge, before a result of the first analysis process is given, whether or not to perform the second analysis process on a basis of a result of the short-time analysis process.

In this invention, before the result of the first analysis process to a sample is obtained, it is judged whether or not to perform the second analysis process. Therefore, when it is judged that the second analysis process is to be performed, the first analysis process to a sample and the second analysis process to the sample are performed simultaneously. Therefore, according to this invention, in cases where a plurality of analysis processes are performed to the sample, it is possible to improve overall performance.

The analysis device according this invention may include a second judging unit different from the judging unit that serves as a first judging unit. The second judging unit judges whether or not to perform the second analysis process on a basis of the result of the first analysis process. In this case, the first judging unit can judge whether or not to perform the second analysis process to the sample earlier than the second judging unit. Moreover, by judging on the basis of both results of the short-time analysis process and the first analysis process whether or not to perform the second analysis process, it becomes possible to determine the necessity of performing the second analysis to the analysis target analyte more accurately.

Further, the analysis device according to the invention may perform the second analysis process when, after the first judging unit judges that the second analysis process to the sample is not to be performed, the second judging unit judges that the second analysis process to the same sample is to be performed. According to this, it is possible to perform the second analysis process on the basis of the result of the short-time analysis process even if the necessity of performing the second analysis process cannot be determined accurately.

Here, at least part of analysis items measured in the short-time analysis process may be identical with or relevant to part of analysis items measured in the first analysis process. Further, the part of the analysis items measured in the first analysis process may be measured in the short-time analysis process in a way different from that of the first analysis process. An analysis item measured in the short-time analysis process may differ from analysis items measured in the first analysis process. In every case, it is possible to determine the necessity of performing the second analysis to the analysis target analyte more accurately.

In the analysis device according to the present invention, the short-time analysis unit may be fixed on a nozzle for getting a sample from a sample container, a flow channel through which the sample to be delivered to the first analysis unit flows, or a flow channel through which the sample to be delivered to the second analysis unit flows. With this configuration, it is possible to perform the short-time analysis process to a sample before the sample is supplied to the second analysis unit.

The analysis device according to the invention may include a sample reservoir capable of temporarily storing the sample the second analysis process to which has been performed and the second analysis process to which has not been performed. In this case, the sample is delivered from the sample reservoir to the second analysis unit when the second analysis process is performed. Thus, by storing the obtained sample in the sample reservoir, it becomes possible to perform the second analysis process, which is judged to be performed, without re-obtaining the sample from the sample container.

Here, the sample reservoir may be united with the short-time analysis unit. With these configurations, the short-time analysis process can be performed using the sample stored in the sample reservoir.

An analysis device according to the fourth invention is a device of performing a predetermined analysis processing to a sample. The analysis device includes: a first analysis unit and a second analysis unit to perform a first analysis process to the sample and a second analysis process to the sample, respectively; and a sample reservoir capable of temporarily storing the sample to be delivered to the second analysis unit.

According to this invention, when the second analysis process to a sample is performed in the second analysis unit, the sample in the sample reservoir is supplied to the second analysis process. Therefore, by analyzing samples in a way that the sample is stored in the sample reservoir, it is possible to advance the first analysis process independent of progress state of the second analysis process. That is, immediatelyafter the first analysis process to the current sample is completed, the first analysis process to the next sample can be performed. Therefore, it is possible to improve performance of overall analysis processing.

The analysis device according to this invention may include a nozzle to get from a sample container the sample to be delivered to the first analysis unit and the second analysis unit. In this case, it is possible to supply the gotten sample to the first analysis unit and to store the gotten sample in the sample reservoir at the first stage.

Here, in an analysis apparatus that has not the sample reservoir, when performing the second analysis process to a sample, the first analysis process to which is completed, it is necessary to re-get the sample from the sample container by the nozzle. However, in the status where multiple samples are conveyed one by one, to re-get a sample, it is necessary to move the nozzle to a position over the sample container in which the sample is stored. Therefore, it is difficult to re-get the same sample with one nozzle after a while. On the other hand, the analysis unit according the present invention includes the sample reservoir. Therefore, as mentioned above, when performing the second analysis process to a sample, the sample can be supplied from the sample reservoir to the second analysis unit. In other words, when performing the second analysis process to the sample the first analysis process to which is completed, it is not necessary to re-get this sample from the sample container by the nozzle. Accordingly, use of the sample reservoir facilitates sharing of the nozzle among the analysis units.

Sharing of the nozzle by the analysis units enables sharing of part of flow channels that supplies the analyte to each analysis unit, and sharing of a cleaning unit that cleans the nozzle. Consequently, it is possible to miniaturize the analysis device. Moreover, it is possible to cut production costs of the analysis device.

Note that, in cases where the nozzle is shared between the first analysis unit and the second analysis unit, it is possible to supply immediately after completion of the first analysis process the next sample to the first analysis unit. Therefore, it is possible to improve efficiency of overall analysis processing.

The analysis unit according the present invention may further include judging means for judging whether or not to perform the second analysis process on a basis of a result of the first analysis process. In this case, the second analysis process to a sample is performed after the first analysis process to the sample is completed. According to the analysis unit of the present invention, also in such a case, it is possible to supply immediately after completion of the first analysis process the next sample to the first analysis unit. Therefore, it is possible to improve efficiency of overall analysis processing.

In the analysis device according to the invention, the sample reservoir may include an internal reservoir fixed on a flow channel through which the sample to be delivered to the second analysis unit flows. The analysis device according to this invention may further include: a first flow channel through which the sample to be delivered to the first analysis unit flows; a second flow channel through which the sample to be delivered to the second analysis unit flows; and a switching valve to supply the sample gotten by the nozzle to either the first flow channel or the second analysis unit, and may include the sample reservoir including an internal reservoir fixed on the second flow channel.

With these configurations, it is possible to store the sample that is gotten by the nozzle in the sample reservoir, and is not yet supplied to the second analysis unit into the internal reservoir. And when performing the second analysis process, the sample stored in the internal reservoir can be supplied to the second analysis unit through the flow channel which leads to the second analysis unit.

Further, in the analysis device according to this invention, which has the nozzle gets the sample by sucking the sample from the sample container, the sample reservoir may include an external reservoir to store the sample discharged from the nozzle, the sample reservoir being arranged at a position where the nozzle is able to discharge the sucked sample therein. In this case, the sample may be sucked from the external reservoir by the nozzle when the sample is delivered to the second analysis unit.

With this configuration, it is possible to store the sample that is gotten by the nozzle in the sample reservoir, and is not yet supplied to the second analysis unit into the external reservoir. And when performing the second analysis process, the sample stored in the external reservoir can be supplied to the second analysis unit through the flow channel which leads to the second analysis

Note that, the sample reservoir according the present invention may have both of the internal reservoir and the external reservoir.

Moreover, in the analysis unit according to the first and the second invention, the first and second analysis units may be placed in a housing. According to this, it is possible to miniaturize the analysis device.

An analysis system according to the first invention is a system to perform a specific analysis process to a sample. The analysis system includes: a first analysis device, a second analysis device, and a short-time analysis device to perform a first analysis process to the sample, a second analysis process to the sample, and a short-time analysis process to the sample, by which an analysis result is given in a shorter time than by the first analysis process, respectively; a judging device to judge whether or not to perform the second analysis process on a basis of a result of the first analysis process before a result of the first analysis process is given.

That is, the analysis system according to the first invention, when supplying the same sample to each of the analysis devices, operates as a system where the second analysis process to a sample can be started before completion of the first analysis process to the same sample. Therefore, also by this analysis system, it is possible to improve efficiency of analysis processing including two or more analysis processes to a sample.

An analysis system according to the second invention is a system to perform a specific analysis process to a sample. The analysis system includes: a first analysis device and a second analysis device to perform a first analysis process to the sample and a second analysis process to the sample, respectively; and a sample reservoir capable of temporarily storing the sample to be delivered to the second analysis device.

According to the analysis system of this invention, effect similar to the effect achieved by the analysis device according to the second invention can be achieved.

An analysis method according to the first invention is a method of performing a specific analysis process to a sample. The analysis method includes: a first analysis step, a second analysis step, and a short-time analysis step of performing a first analysis process to the sample, a second analysis process to the sample, and a short-time analysis process, by which an analysis result is given in a shorter time than by the first analysis process, respectively; and a judging step of judging whether or not to perform the second analysis process on a basis of a result of the first analysis process before a result of the first analysis process is given.

The analysis method according to this invention may include the judging step serves as a first judging step and further include a second judging step of judging whether or not to perform the second analysis process on a basis of the result of the first analysis process.

In the analysis method according to this invention, the second analysis process may be performed when, after the first judging step judges that the second analysis process to the sample is not to be performed, the second judging step judges that the second analysis process to the same sample is to be performed.

In the analysis method according to this invention, as with the analysis device according to the present invention, at least part of analysis items measured in the short-time analysis process may be identical with or relevant to part of analysis items measured in the first analysis process. Further, the part of analysis items measured in the first analysis process may be measured in the short-time analysis process in a way different from that of the first analysis process. Moreover, an analysis item measured in the short-time analysis process may differ from analysis items measured in the first analysis process.

Further, the analysis method according to the present invention may further include a sample storing step of storing a sample reservoir with the sample that has been subjected to the simple analysis process and is not subjected to the second analysis process. In this case, the second analysis process may be performed by using the analyte stored in the sample reservoir in the second analysis step.

According to the analysis method of this invention, effect similar to the effect achieved by the analysis device according to the first invention can be achieved.

An analysis method according to the second invention is a method of performing a specific analysis process to a sample. The analysis method includes: a first analysis step and a second analysis step of performing a first analysis process and a second analysis process, respectively; and a sample storing step of temporarily storing the sample in a sample reservoir before the second analysis process is performed in the second analysis step.

Moreover, the analysis method according to this invention may further include a judging step of judging, on a basis of a result of the first analysis process, whether or not to perform the second analysis process.

According to the analysis method of this invention, effect similar to the effect achieved by the analysis device according to the second invention can be achieved.

A program according to the first invention is a program for a computer that controls an analysis device to perform a specific analysis process to a sample, the analysis device including a first analysis unit, a second analysis unit, and a short-time unit to perform a first analysis process to the sample, a second analysis process to the sample, and a short-time analysis process to the sample, which is completed in a shorter time than a first analysis process to the sample, respectively. The program makes the computer execute: a judging step of judging whether or not to perform the second analysis process on a basis of a result of the first analysis process before a result of the first analysis process is given.

The program according to the present invention may make the computer further execute a second judging step of judging whether or not to perform the second analysis process on a basis of a result of the first judging step (the judging step).

The program according to this invention may make the computer execute a step of making the second analysis unit start the second analysis process when, after the first step judges that the second analysis process to the sample is not to be performed, the second step judges that the second analysis process to the same sample is to be performed.

According to the program of the present invention, effect similar to the effect achieved by the analysis device according to the first invention can be achieved.

A program according to the second invention is a program for a computer that controls an analysis device to perform a specific analysis process to a sample, the analysis device including a first analysis unit and a second analysis unit to perform a first analysis process to the sample and a second analysis process to the sample, respectively, and a sample reservoir capable of storing the sample. The program makes the computer execute: a sample storing step of temporarily storing the sample in the sample reservoir before the second analysis process is performed in the second analysis step.

The program according to the present invention may make the computer further execute a judging step of judging whether or not to perform the second analysis process on a basis of a result of the first analysis process.

According to the program of the present invention, effect similar to the effect achieved by the analysis device according to the second invention can be achieved.

The recording medium according to the first invention or the second invention is a medium stored with the above-mentioned program according to the present invention.

### Advantageous Effects of Invention

According to the present invention, in performing two or more analysis processes to a sample, it is possible to improve performance of the overall analysis processing.

### Brief Description of Drawings

FIG. 1 is a block diagram illustrating a configuration of a urine analysis device according to the first embodiment;
FIG. 2 is a block diagram illustrating an internal configuration of the urine analysis device according to the first embodiment;
FIG. 3 is a block diagram illustrating a configuration of the control unit according to the first embodiment;
FIG. 4 is a flowchart showing a flow of the analysis process in the urine analysis device according to the first embodiment;
FIG. 5 is a block diagram illustrating a configuration of a urine analysis device according to a first modified embodiment of the first embodiment;
FIG. 6 is a block diagram illustrating a configuration of a urine analysis device according to a secondmodified embodiment of the first embodiment;
FIG. 7 is a block diagram illustrating a configuration of a urine analysis device according to a third modified embodiment of the first embodiment;
FIG. 8 is a block diagram illustrating a configuration of a urine analysis device according to a second embodiment;
FIG. 9 is a block diagram illustrating a configuration of a control unit according to a second embodiment;
FIG. 10 is a first flowchart showing a flow of the analysis process in the urine analysis device according to the second embodiment;
FIG. 11 is a second flowchart showing a flow of the analysis process in the urine analysis device according to the second embodiment;
FIG. 12 is a block diagram illustrating a configuration of a urine analysis device according to a first modified embodiment of the second embodiment;
FIG. 13 is a block diagram illustrating a configuration of a urine analysis device according to a second modified embodiment of the second embodiment;
FIG. 14 is a block diagram illustrating a configuration of a urine analysis device according to a third modified embodiment of the second embodiment;
FIG. 15 is a block diagram illustrating a configuration of an urine analysis device according to a third embodiment; and
FIG. 16 is a block diagram illustrating a configuration of a control unit according to a fourth embodiment.

### Description of Embodiments

Embodiments of the present invention will hereinafter be described in depth with reference to the drawings. Dimensions, material, shape, relative position, etc. of each component of the embodiments discussed below are not to be construed as limitations of the scope of the invention unless stated otherwise.

Note that, in this specification, the present invention is described by exemplifying a case where the present invention is applied to a urine analysis device to perform a component analysis. The present invention is, however, applicable to device other than the urine analysis device, such as a blood analysis unit to perform the component analysis of blood, etc. Moreover, the following explanation of each embodiment of the analysis device according to the present invention serves also as explanation of embodiments of an analysis device, an analysis system, an analysis method, a program and a recording medium stored with a program according to the present invention.

### <FIRST EMBODIMENT>

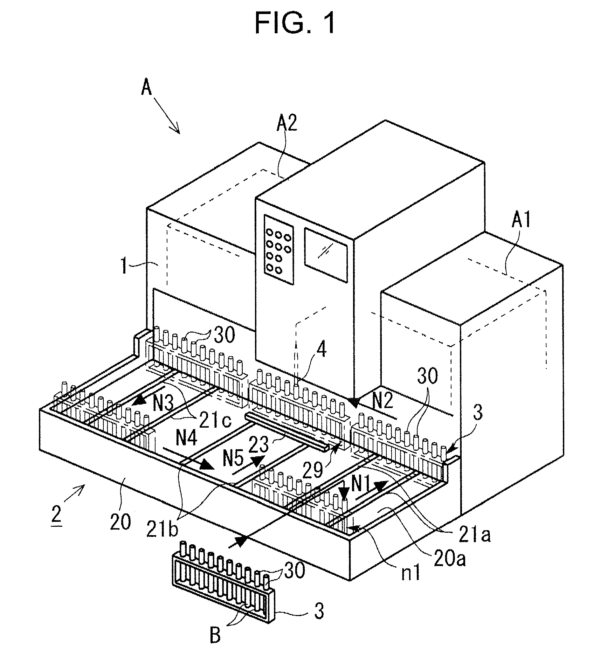

An analysis device according to a first embodiment will be described with reference to FIGS. 1 through 7.

### (CONFIGURATION OUTLINE)

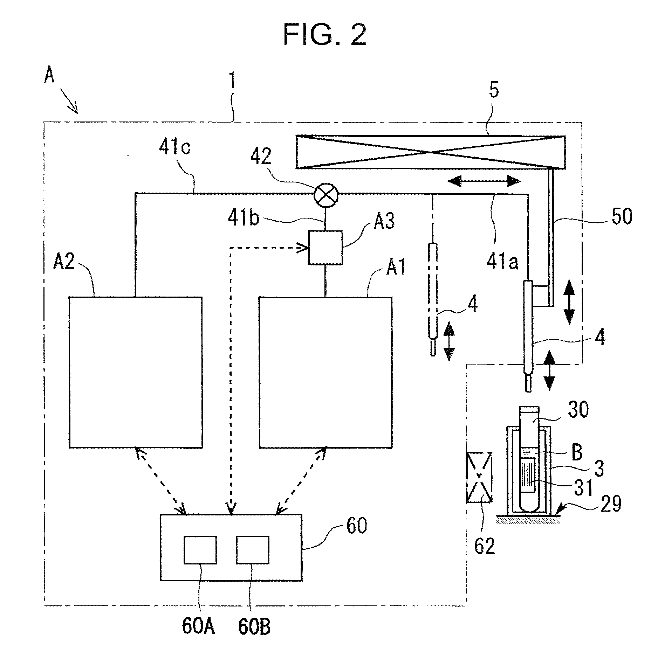

FIGS. 1 and 2 are diagrams each illustrating a configuration of the urine analysis device according to this embodiment. FIG. 1 is an outer view of the device, and FIG. 2 is a block diagram illustrating an internal configuration of the device.

As shown in FIGS. 1 and 2, the urine analysis device according to the present embodiment includes a first analysis unit A1 and a second analysis unit A2 placed in a housing 1. The first analysis unit A1 and the second analysis unit A2 performs their respective analysis processes, which are different from each other, to an analyte to be analyzed. Hereinafter, the analysis process performed by the first analysis unit A1 will be termed the first analysis process, and the analysis process performed by the second analysis unit A2 will be termed the second analysis process.

Specifically, the first analysis process is a urine qualitative test, and the second analysis process is a urine sediment test. In the urine qualitative test, concentrations of protein, glucose, hemoglobin, bilirubin and the like in urine are measured. In the urine sediment test, one or more concrete components (particles) contained in urine, such as blood corpuscle, epithelial cell, bacillus, crystal, etc., are measured. In this way, analysis items and analysis method of the second analysis process differ from those of the first analysis process.

Note that, as the practical configuration of the first analysis unit A1, any conventional configuration capable of performing the urine qualitative test can be adopted. Further, as the practical configuration of the second analysis unit A2, any conventional configuration capable of performing the urine sediment test can be adopted. In this way, analysis items and analysis method of the second analysis process differ from those of the first analysis process. For instance, it is possible to adopt, as the first analysis unit A1, a unit to make chemical measurement (e.g. measurement which uses test papers), and to adopt, as the second analysis unit A2, a unit to perform the urine sediment test (quantitative measurement of particles in urine) employing flow cytometry.

The urine analysis device A includes a carrier system 2. The carrier system 2 is a system for carrying spitzes 30 each containing urine B that is an analyte (which corresponds to a sample of the present invention). Note that, although the round bottom test tube is depicted as the spitz 30 in each Figure for convenience in illustration, the spitz 30 is defined as the conical bottom test tube. Of course, a container for urine B (sample) can be chosen appropriately, and it is not required to limit the shape of the container to a particular shape. The carrier system 2 carries the spitzes 30 in the condition where they are held upright in an analyte rack 3. The carrier system 2 includes a frame 20 concatenated with the lower front part of the housing 1, three rotatably drivable belts 21a through 21c positioned on the upside part 20a of the frame 20, and two pusher (not shown) movable in the horizontal direction.

In the carrier system 2, when the analyte rack 3 is set at the position indicated by the symbol n1, the analyte rack 3 is carried in the direction of arrow N1, and then is carried by the pusher in the direction indicated by arrow N2. Next, the analyte rack 3 is carried by the belt 21c in the direction of arrow N3, and then is carried by the pusher in the direction indicated by arrow N4 onto the belt 21b.

The urine analysis device A further includes a nozzle 4 to obtain urine B to be analyzed from the spitz 30 that has been carried by the carrier system 2. The nozzle 4 is placed at a position over the carrying path 29 along which the analyte rack 3 is carried in the direction of arrow N2. The nozzle 4 sucks urine B from the spitz 30 held by the analyte rack 3 positioned on the carrying path 29. Thus, the nozzle 4 obtains urine B to be analyzed.

As shown in FIGS. 1 and 2, the nozzle 4 is the only nozzle arranged in the urine analysis device A according to the present embodiment to obtain the analyte. That is, each of the analyte to be supplied to the first analysis unit A1 and the analyte to be supplied to the second analysis unit A2 is obtained by the nozzle 4. Sharing of the nozzle by the first and second analysis units A1 and A2 enables sharing of part of flow channels that supply the analyte to the analysis units A1 and A2, and sharing of a cleaning unit that cleans the nozzle 4. Consequently, it is possible to miniaturize the urine analysis device A. Moreover, it is possible to cut production costs of the urine analysis device A.

Herein, the internal configuration of the urine analysis device A is discussed referring to FIG. 2. Within the urine analysis device A, other than the first and second analysis units A1 and A2, a nozzle moving device 5, urine flow channels 41a, 41b and 41c, a three-way valve 42, a simple analysis unit A3, and a control unit 60 are provided.

The nozzle moving device 5 includes an arm 50 that supports the nozzle 4. The nozzle moving device 5 moves, by moving the arm 50, the nozzle 4 in the vertical direction and the horizontal direction (the directions of arrows in FIG. 2).

To the nozzle 4, one end of a common urine flow channel 41a is connected. The other end of the common urine flow channel 41a is connected to the three-way valve 42. To the three-way valve 42, one end of each of the first and second urine flow channel 41b and 41c is connected. The three-way valve 42 is capable of flowing urine B obtained by the nozzle 4 from the common urine flow channel 41a to either of the first and second urine flow channel 41b and 41c.

Further, the other end of the first urine flow channel 41b is connected to the first analysis unit A1, and the other end of the second urine flow channel 41c is connected to the second analysis unit A2. Namely, urine B obtained by the nozzle 4 is supplied to the first analysis unit 41A through the common urine flow channel 41a and first urine flow channel 42b, and is supplied to the second analysis unit 41B through the common urine flow channel 41a and the second urine flow channel 42c.

Furthermore, the simple analysis unit A3 is arranged on an intermediate part of the first urine flow channel 41b. This simple analysis unit A3 performs a simple analysis process to the analyte to be analyzed (to the analyte that passes through the simple analysis unit A3). In the simple analysis process by the simple analysis unit A3, one or more analysis items that are relevant to part of the analysis items measured in the first analysis process of the first analysis unit A1 (that is, the analysis items measured in the urine qualitative test) are measured using a method simpler than the measurement method of the first analysis process.

Examples of the simple analysis process are measurement of turbidity of urine using the transmitted scattered light method, measurement of degree of red color of urine, measurement of urine color, etc. The turbidity of urine has relation to the concentration of protein, and the degree of red color of urine has relation to the concentration of hemoglobin. Further, the urine color has relation to the concentrations of various components of urine that are measured in the urine qualitative test. Analysis time per one analyte of each of the simple analysis processes like these is extremely shorter compared to that of the first analysis process.

Note that, in the simple analysis process, it is possible to measure one or more analysis items that are identical to part of the analysis items measured in the simple analysis process of the simple analysis unit A3. For example, the specific gravity of urine may be measured on the basis of the refractive index of urine. Moreover, in the above-mentioned instantiation, concentration of protein may be computed from the turbidity of urine, and the concentration of hemoglobin may be computed from the degree of red color of urine. Further, in the case where one or more analysis items that are measured in the urine qualitative test, these analysis items can be removed from the analysis items measured in the first analysis process. In this case, the analysis items of the first analysis process differ from those of the simple analysis process. In any case, in the simple analysis process, one or more analysis items are measured using a method simpler than the measurement method of the first analysis process. Accordingly, analysis time per one analyte of the simple analysis process is extremely shorter compared to that of the first analysis process.

The control unit 60 is a computer including a CPU 60A, a memory 60B, etc. It includes a volatile memory and a non-volatile memory as the memory 60B (a recording media), and the non-volati le memory is stored with a computer program (which will hereinafter also simply be referred to as the program). The CPU (Central Processing Unit: microprocessor) 60A reads the program into the volatile memory when the analysis device A is started (turned on), and then executes the program.

The control unit 60 is electrically connected to the first analysis unit A1, the second analysis unit A2, the simple analysis unit A3, the nozzle moving device 5, the three-way valve 42, etc. The control unit 60 the CPU 60A of which is executing the above-mentioned program (hereinafter simply be termed the control unit 60) performs data processing relating to analysis results of the analysis units A1, A2 andA3, and controls operation of each unit (or device) mentioned above. Thus, in the present embodiment, the overall operation of the urine analysis device A is controlled by one control unit 60.

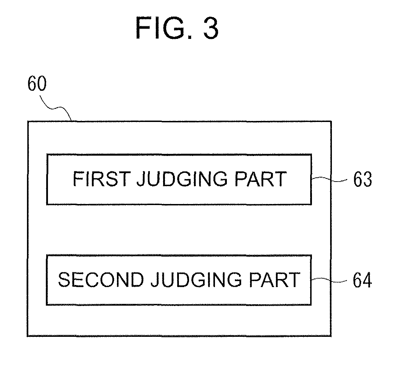

As illustrated in FIG. 3, the control unit 60 is provided with a first judging part 63 and a second judging part 64 that are functional parts. Function of each judging part 63, 64 will be discs sued later.

Note that, as shown in FIG. 2, an identification code 31, suchasabarcode, is attached to the spitz 30. The urine analysis device A includes a reading unit 62 to read the identification code 31. The reading unit 62 is electrically connected to the control unit 60, and identification data read by the reading unit 62 is inputted to the control unit 60. The inputted identification data is used as reference data that is associated with analysis result data on urine B obtained by each analysis unit A1, A2 or A3.

Further, within the urine analysis device A, though not illustrated, a cleaning unit to clean the nozzle 4 is provided. The cleaning unit includes a cleaning fluid tank to store cleaning fluid, a feeding device to feed cleaning fluid from the cleaning fluid tank into the nozzle 4, and a waste fluid tank to store waste fluid that is cleaning fluid discharged, after being used to clean the nozzle 4, from the nozzle 4.

In the present embodiment, the first and second analysis unit A1 and A2 respectively correspond to the first and second analysis unit of each of the analysis devices according to the first through third invention, the simple analysis unit A3 corresponds to the short-time analysis unit of each of the analysis devices according to the first through third invention. Further, the first judging part 63 of the control unit 60 corresponds to the judging unit or the first judging unit of each of the analysis devices according to the first through third invention, and the second judging part 64 of the control unit 60 corresponds to the second judging unit of the analysis devices according to the fourth invention.

### (FLOW OF ANALYTICAL PROCESS)

In the urine analysis device A, the first analysis process to an analyte to be analyzed is performed by the first analysis unit A1 before the second analysis process to the analyte is performed by the second analysis unit A2. Subsequently, it is judged, on the basis of the result of the first analysis process, whether or not the second analysis process to the analyte is to be performed. When it is judged that the second analysis process is to be performed, analyte identical with the analyzed analyte is supplied to the second analysis unit A2, and then the second analysis process is performed.

However, when the second analysis process to an analyte is not performed until the result of the first analysis process to the analyte is obtained, there is a possibility that the overall analysis performance degrades. Accordingly, in the urine analysis device A, before the result of the first analysis process is obtained, it is judged, on the basis of the result of the simple analysis process, whether or not the second analysis process to the analyte is to be performed.

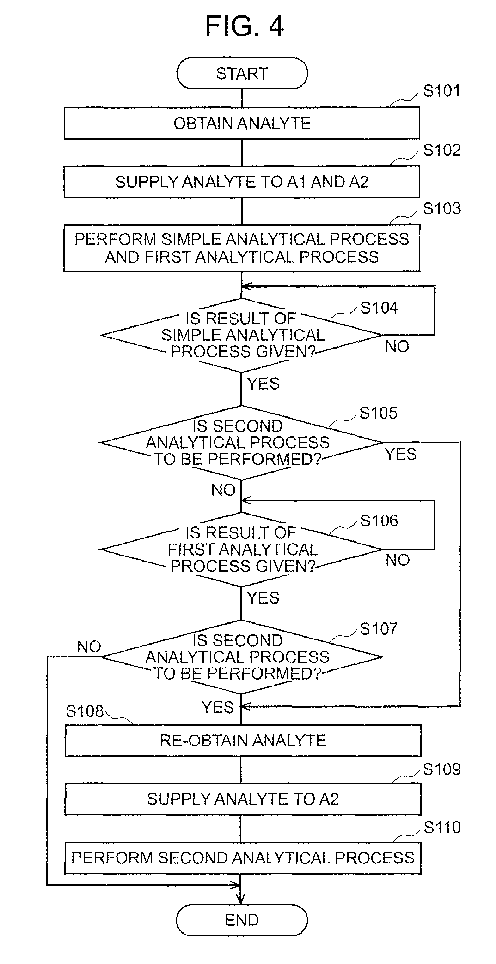

Flow of the analysis process will be described with reference to the flowchart shown in FIG. 4. Note that this flow of the analysis process is executed by the control unit 60 (the CPU 60A in the control unit 60) in accordance with the program on the non-volatile memory.

In the flow of this analysis process, first, an analyte to be analyzed is obtained by the nozzle 4 at step S101. Next, the obtained analyte is delivered to the simple analysis unit A3 and the first analysis unit A1 at step S102. Then, at step S103, the simple analysis process to the delivered analyte is performed by the simple analysis part A3, and the first analysis process to the delivered analyte is performed by the first analysis part A1. Note that, in the present embodiment, this step S103 corresponds to the first analysis step and the short-time analysis step of the analysis method according to the first invention.

Then, at step S104, it is judged whether or not the result of the simple analysis process by the simple analysis unit A3 is obtained. It should be noted that the result of the simple analysis processes can be obtained within a short period of time after the process is started since analysis time of the simple analysis process is very short as mentioned above. When the result of the simple analysis process is obtained, it is judged, at step S105, whether or not the second analysis process to the same analyte is to be performed. In the present embodiment, the first judging part 63 of the control unit 60 performs this step S105 of judging whether or not the second analysis process is to be performed. Note that, in the present embodiment, this step S105 corresponds to the judging step or the first judging step of the analysis method according to the first invention.

Thereafter, when it is judged, based on the result of the simple analysis process, that the second analysis process to the analyte is to be performed, the second analysis process to the analyte to be analyzed is started. In this case, the analyte to be analyzed is obtained again from the spitz 30 by the nozzle 4 at step S108. Next, the obtained analyte is supplied to the second analysis unit A2 at step S109. Then, the second analysis process is performed to the analyte supplied to the second analysis unit A2 at step S110.

On the other hand, when it is judged, based on the result of the simple analysis process, that the second analysis process is not to be performed, it is judged, at step S107, whether the result of the first analysis process by the first analysis unit A1 is obtained. If the result of the first analysis process by the first analysis unit A1 is not yet obtained, process goes into wait state until the result is obtained. When the result of the first analysis process is obtained, it is re-judged, at step S107, whether or not the second analysis process to the analyte is to be performed. In the present embodiment, the first judging part 63 of the control unit 60 performs this step S107 of judging whether or not the second analysis process is to be performed. Incidentally, in the present embodiment, this step S105 corresponds to the second judging step of the analysis method according to the first invention

In the case where the second analysis process is judged to be performed on the basis of the result of the simple analysis process, the second analysis process to the analysis target analyte is performed according to steps S108 through S110 described above. On the other hand, In the case where the second analysis process is judged not to be performed on the basis of the result of the simple analysis process, the second analysis process to the analyte is not performed.

According to the above-mentioned analysis process flow, it is possible to perform the first analysis process to an analyte and the second analysis process to the analyte in the same period of time when the second analysis process is determined to be performed on the basis of the result of the simple analysis process. It is therefore possible to improve overall performance of the analysis process.

Moreover, according to the above-mentioned flow, even if the second analysis process is determined to be performed on the basis of the result of the simple analysis process, whether the second analysis process is to be performed or not is re-judged on the basis of the result of the first analysis process. Thereafter, when the second analysis process is judged to be performed as a result of re-decision, the second analysis process to the analyte is performed. Consequently, it is possible to determine the necessity of performing the second analysis to the analysis target analyte more accurately. Furthermore, it is possible to perform the second analysis process even if the necessity of performing the second analysis process cannot be determined accurately.

Note that it is not necessary to determine whether the second analysis process to be performed to each and every analyte. For instance, to the analyte that is designated to perform the second analysis process thereto regardless of the result of the first analysis process, the first analysis process and second analysis process may be performed simultaneously. Moreover, the simple analysis process to the analyte that is designated to perform the second analysis process regardless of the result of the first analysis process may be omitted.

Computer program to realize the analysis process flow shown in FIG . 4 can be stored in a recording media readable by a computer (control unit 60). Further, the flow can be realized by making a computer read and execute the program.

Herein, the computer readable recording medium connotes a recording medium capable of storing information such as data and programs electrically, magnetically, optically, mechanically or by chemical action, which can be read from the computer. Among these recording mediums, those demountable from the computer are, for example, a flexible disk, a magneto-optic disc, a CD-ROM, a CD-R/W, a DVD, a DAT, an 8mm tape, a memory card. Further, a hard disk, a ROM (Read-Only Memory) and the like are given as the recording mediums fixed within the computer.

### (MODIFIED EXAMPLES)

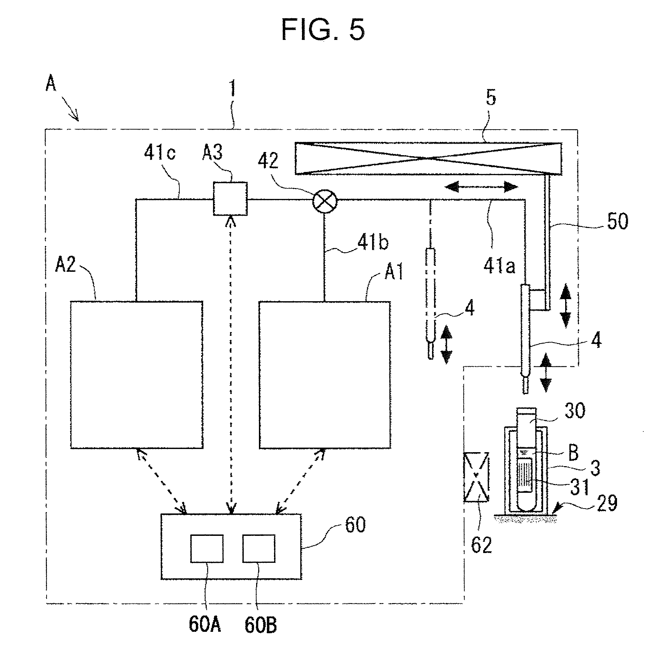

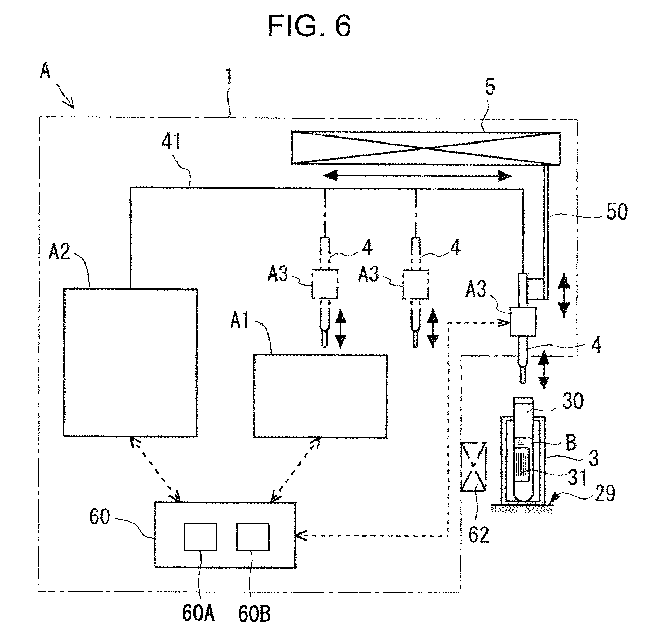

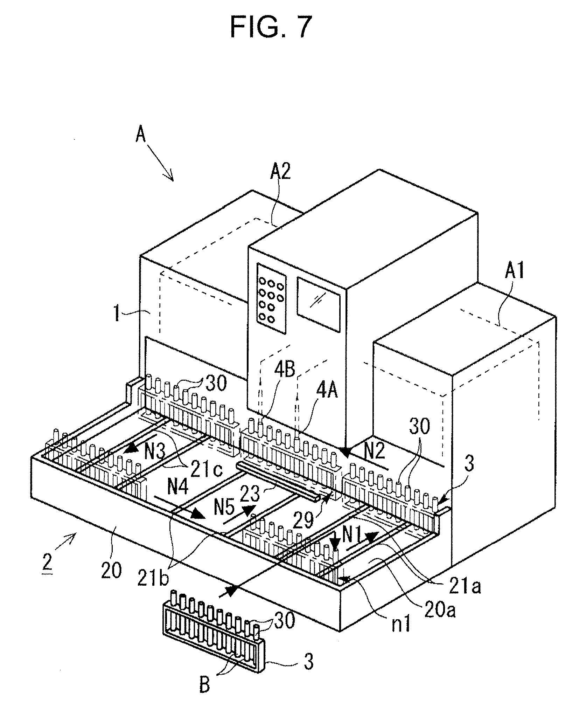

Configuration of urine analysis devices according to modified examples of the first embodiment will be described referring to FIGS. 5 through 7. FIG. 5 is a block diagram illustratingconfiguration ofan urine analysis device according to a first modified example. FIG. 6 is a block diagram illustrating configuration of an urine analysis device according to a second modified example. FIG. 7 is a block diagram illustratingconfiguration ofan urine analysis device according to a third modified example. Note that explanations of parts same with the aforementioned parts will be omitted.

As shown in FIG. 5, the simple analysis unit A3 of the urine analysis device A according to the first modified example is arranged on an intermediate part of the second urine flow channel 41c. With this configuration, when the simple analysis process to some analyte is performed and the second analysis process to the analyte is judged to be performed, the analyte within the simple analysis unit A3 can be supplied to the second analysis unit A2. It is therefore not necessary to re-obtain the analyte to be supplied to the second analysis unit A2 from the spitz 30 by the nozzle 4. The first modified example will be described in more detail. As illustrated in FIG. 5, the urine analysis device A includes the simple analysis unit A3 as with the urine analysis device according to the first embodiment. However, the simple analysis unit A3 of the urine analysis device A according to the first modified example is arranged not on an intermediate part of the second urine flow channel 41b, but on an intermediate part of the second urine flow channel 41c. Moreover, the simple analysis unit A3 is a unit capable of holding in its inside the analyte of a quantity required for the second analysis process by the second analysis part A2. Furthermore, the simple analysis unit A3 has ability to discharge the analyte therein toward the second analysis unit A2 when a fixed instruction is inputted from the control unit 60.

The urine analysis device A according to the first modified example includes the three-way valve 42 capable of divide urine B obtained by the nozzle 4 among the first urine flow channel 41b and second urine flow channel 41c. Note that the three-way valve 42 is a unit prepared for the purpose of allowing urine B to be supplied only toward the first urine flow channel 41b (allowing only the first analysis process to urine B can be performed) and allowing urine B to be supplied only toward the second urine flow channel 41b (allowing only the second analysis process to urine B to be performed). Accordingly, in the case where both of the first analysis process and the simple analysis process are always performed, it is possible to remove the three-way valve 42 (to connect the urine flow channels 41a through 41c with each other directly).

Further, in the urine analysis device A according to the first modified example, every time analyte is analyzed, each part, under the control of the control unit 60, operates as follows.

At the time of analysis of a certain analyte, first, the required amount of analyte for the both of the first analysis process and the second analysis process is obtained from the spitz 30 by the nozzle 4, and part of the obtained analyte (the required amount of analyte for the first analysis process) is supplied to the first analysis unit A1 through the first urine flow channel 41b, etc. Further, at the same time, the rest of the obtained analyte (the required amount of analyte for the second analysis process) is supplied to the second analysis unit A1 through the second urine flow channel 41c, etc.Then, the simple analysis process by the simple analysis unit A3 and the first analysis process by the first analysis unit A1 are initiated.

As already explained, the simple analysis process is the process whose analysis time per one analyte is extremely shorter compared to that of the first analysis process. Therefore, the simple analysis process is completed before the first analysis process is completed.

On completion of the simple analysis process, it is judged by the control unit 60 whether or not the current analysis target analyte is to be subjected to the second analysis process on the basis of information (refractive index, specific gravity, turbidity and so on) acquired by the simple analysis process. When the current analysis target analyte is the analyte that is to be subjected to the second analysis process, the analyte within the simple analysis process A3 is supplied to the second analysis unit A2, and then the second analysis process to the analyte is started. Note that, in the case where the current analysis target analyte is not the analyte that is to be subjected to the second analysis process, the analyte within the simple analysis process A3 is discarded by a device which is not illustrated in the Figure.

As described above, this urine analysis device A has such configuration that the first analysis process and the second analysis process are performed by obtaining analyte only once from the spitz 30. On the other hand, the above-mentioned urine analysis device A according to the first embodiment is the device that obtains analyte twice from the spitz 30. Consequently, it follows that this urine analysis device A according to the first modified example is the device where time required to complete the second analysis process is shorter than that of the urine analysis device A according to the first embodiment.

The urine analysis device A according to the second modified example includes the first analysis unit A1 that dispenses a drop of urine to be analyzed on a test strip, and performs urine qualitative test using the test strip. Therefore, as shown in FIG. 6, the nozzle 4 is configured so as to move to the position over a spot application part (not shown) of the first analysis unit A1. The analyte obtained by the nozzle 4 is discharged to the test strip set on the spot application part.

In this modified example, because the analyte is supplied to the first analysis unit A1 as mentioned above, only one urine flow channel 41, which connects between the nozzle 4 and the second analysis unit A2, is provided. Further, the simple analysis unit A3 is attached to the nozzle 4.

With this configuration, the analyte obtained by nozzle 4 can be subjected to the simple analysis process in the simple analysis unit A3. And, when the second analysis process to the analyte is determined to be performed on the basis of the result of the simple analysis process, the analyte within the nozzle 4 can be supplied to the second analysis unit A2. Consequently, with this configuration, it is also not necessary to re-obtain the analyte to be supplied to the second analysis unit A2 from the spitz 30 by the nozzle 4.

The secondmodified example will be discussed in more detail. The urine analysis device A (FIG. 6) according to the second modified example is a modified version of the urine analysis device A (FIG. 5) according to the first modified example. Specifically, the urine analysis device A according to the second modified example includes the first analysis unit A1 that dispenses a drop of urine to be analyzed on a test strip, and performs the urine qualitative test using the test strip. Therefore, as shown in FIG. 6, the urine analysis device A according to the second modified example is so configured that the nozzle 4 can move, within the urine analysis device, to the position over the spot application part (not shown) of the first analysis unit A1. Further, the urine analysis device A according to the second modified example includes only one urine flow channel 41 connecting between the nozzle 4 and the second analysis unit A2.

Moreover, the simple analysis unit A3 of the urine analysis device A according to the second modified example (which is the same as the simple analysis unit A3 of the urine analysis device A according to the first modified example) is fixed to the nozzle 4. Further, the urine analysis device A according to the second modified example is configured so that, every time analyte is analyzed, each part thereof operates as follows.

To begin with, the required amount of analyte for both of the first analysis process and the second analysis process is obtained from the spitz 30 by the nozzle 4. After obtaining the analyte (in this modified example, while in movement of the nozzle 4), the simple analysis process to the obtained analyte is performed. Further, the nozzle 4 is moved, in a way that overlaps with the simple analysis process, or before or after the simple analysis process, to the spot application part (not shown) of the first analysis device A1, and then part of the obtained analyte is discharged to the test strip set on the spot application part of the first analysis unit A1. Thereafter, the first analysis process by the first analysis unit A1 is started.

When it is found out from the result of simple analysis process that the analyte is to be subjected to the second analysis process, the analyte within the nozzle 4 is supplied to the second analysis unit A2, and the second analysis process to the analyte is performed. That is, the second analysis process can be properly performed by determining whether the analyte is to be subjected to the second analysis process only from the result of the simple analysis (without waiting for the result of the first analysis process). For instance, it is possible to perform the second analysis process, which is determined to be performed, concurrently with the first analysis process.

As apparent from the explanation given above, this urine analysis device A according to the second modified example also has such configuration that the first analysis process and the second analysis process are performed by obtaining analyte only once from the spitz 30. Further, the second analysis process can be performed without waiting for the result of the first analysis process. Consequently, according to this urine analysis device, it is possible to greatly reduce time required to complete the second analysis process.

As shown in FIG. 7, the urine analysis device A according to the third modified example includes two nozzles to obtain analyte from the spitz 30. The analyte to be supplied to the first analysis unit A1 is obtained by the nozzle 4A, and the analyte to be supplied to the second analysis unit A2 is obtained by the nozzle 4B. In this case, the urine flow channel to supply the analyte to the first analysis unit A1 and the urine flow channel to supply the analyte to the second analysis unit A2 are provided independently.

In a configuration similar to the configuration of this modified example, it is preferable that whether or not to perform the second analysis process to the analyte be judged until the spitz 30 from which the analyte was obtained by the nozzle 4A is moved to the position under the nozzle 4B. The reason is that, with this, it follows that only the analyte judged to be subjected to the second analysis process can be obtained by the nozzle 4B. However, if time to the determination of whether or not to perform the second analysis process is long, it will be necessary to lengthen the carry distance of the spitz 30 between the position under the nozzle 4A to the position under the nozzle 4B. Elongating the carry distance between them may cause enlargement of the urine analysis unit A.

Accordingly, in this modified example, the simple analysis unit A3 may be attached to the flow channel to supply the analyte from the nozzle 4A to the first analysis unit A1. With this, whether or not to perform the second analysis process can be judged on the basis of the result of the simple analysis process in the simple analysis unit A3, and therefore it is possible to shorten the carry distance of the spitz 30 between the position under the nozzle 4A to the position under the nozzle 4B. As a result, it becomes possible to miniaturize the urine analysis device A.

There are cases where analyte in the spitz 30 can be obtained by the nozzle 4B before the first analysis process is completed depending on contents of the first analysis process to the analyte, distance between the nozzles 4A and 4B, etc. In such cases, it follows that the simple analysis process to the analyte obtained by the nozzle 4B is completed earlier than the first analysis process. Consequently, in such cases, the simple analysis unit A3 can be attached to the urine flow channel for analyte that is supplied from the nozzle 4B to second analysis unit A2.

Furthermore, it is possible to divide the urine analysis device A according to the third modified example whose simple analysis unit A3 is located near the nozzle 4A into a nozzle 4A-side device (an quantitative analysis device including the nozzle 4A, the simple analysis unit A3, the first analysis unit, etc.) and a nozzle 4B-side device (a sediment test device including the nozzle 4B, the second analysis unit A2, etc.), and to produce a system including the nozzle 4A-side device, the nozzle 4B-side device, and a carrying system connected to both devices, wherein sampling and transportation of spitz are performed at proper timings on the basis of the result of simple analysis process or the racks 3 (or the spitzes 30) of the same contents are supplied to respective devices. Similarly, it is also possible to divide the urine analysis device A according to the third modified example whose simple analysis unit A3 is located near the nozzle 4B into a nozzle 4A-side device (an quantitative analysis device including the nozzle 4A, the first analysis unit A1, etc.) and a nozzle 4B-side device (a sediment test device including the nozzle 4B, the simple analysis unit A3, the second analysis unit A2, etc.), and to produce a system including the nozzle 4A-side device, the nozzle 4B-side device, and a carrying system connected to both devices, wherein sampling and transportation of spitz are performed at proper timings on the basis of the result of simple analysis process or the racks 3 (or the spitzes 30) of the same contents are supplied to respective devices.

### <SECOND EMBODIMENT>

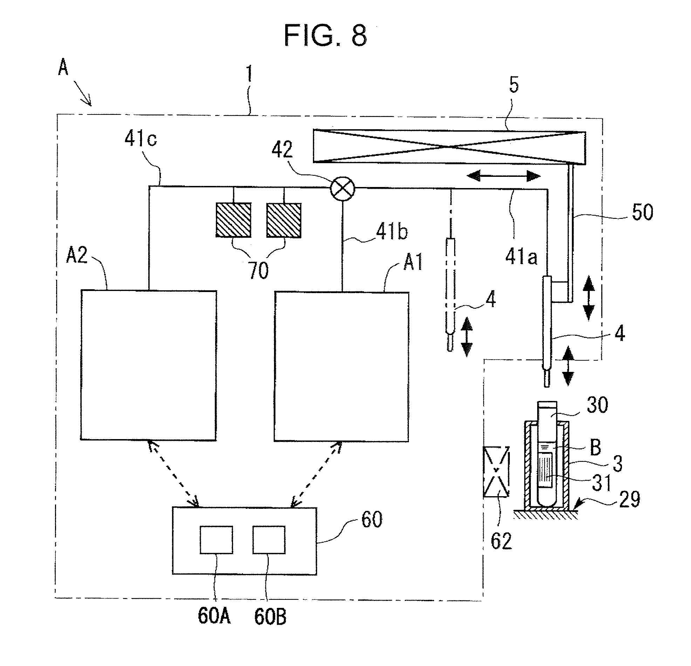

A urine analysis devices according to the second embodiment will be discussed referring to FIGS. 8 through 13. Note that parts that correspond to the conventional technology in this embodiment are the same with that of the analysis device according to the above-mentioned first embodiment. Therefore, the following discussion will be focused on a different parts from those of the urine analysis device according to the first embodiment, and explanations of parts same with the device will be omitted.

### (CONFIGURATION OUTLINE)

FIG. 8 is a block diagram illustrating the urine analysis device according to the present embodiment. Note that the appearance of the urine analysis unit according to the present embodiment is the same with the appearance of the urine analysis device according to the first embodiment shown in Fig. 1. In Fig. 8, the same numerals are employed as those used for the description of the urine analysis device according to the first embodiment in FIG. 5.

The urine analysis device according to the present embodiment includes, as with the first embodiment, the nozzle 4 to obtain urine, which is analyte, from the spitz 30, the first analysis unit A1 and the second analysis unit A2. Contents of analysis in the first and second analysis unit A1 and A2 are the same with those of the first embodiment. However, the urine analysis device according to the present embodiment does not include the simple analysis unit A3 of the first embodiment.

The urine analysis device according to the present embodiment includes a plurality of urine reservoirs each capable of temporarily storing analyte obtained by the nozzle 4. The urine reservoirs are arranged on an intermediate part of the second urine flow channel 41c. The urine reservoirs are electrically connected to the control unit 60. Note that the number of the urine reservoirs is not necessarily need to be larger than one.

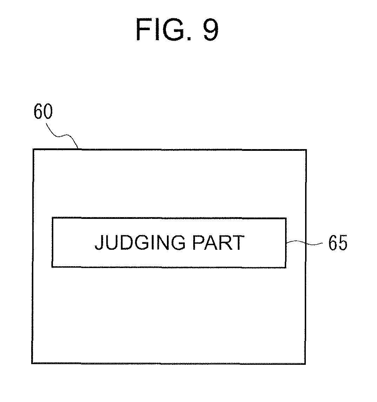

As illustrated in FIG. 9, the control unit 60 is provided with a judging part 65 that is functional parts. Function of the judging part 65 will be discs sued later.

In the present embodiment, the first and second analysis unit A1 and A2 respectively correspond to the first and second analysis unit of the analysis devices according to the second invention. Further, the urine reservoir 70 corresponds to the sample reservoir of the analysis devices according to the second invention. Moreover, the judging part 65 of the control unit 60 corresponds to the judging unit of the analysis device

### according to the second invention.

### (FLOW OF ANALYTICAL PROCESS)

Also in the urine analysis device A according to the present embodiment, the first analysis process to an analyte to be analyzed is performed by the first analysis unit A1 before the second analysis process to the analyte is performed by the second analysis unit A2. Subsequently, it is judged, on the basis of the result of the first analysis process, whether or not the second analysis process to the analyte is to be performed. When it is judged that the second analysis process is to be performed, analyte identical with the analyzed analyte is supplied to the second analysis unit A2, and then the second analysis process is performed.

However, if the first analysis process to the next analysis target analyte cannot be performed until the second analysis process to the current analysis target analyte is completed, there is a possibility that the overall analysis performance degrades. Accordingly, in this embodiment, when the first analysis process is completed, analyte which is the next analysis target is obtained from the spitz 30 by the nozzle 4 even if the second analysis process by the second analysis unit A2 is in progress. Thereafter, the obtained analyte is distributed to the first analysis unit A1 and the urine reservoir 70. The urine reservoir 70 temporarily stores the distributed analyte. When it is judged, on the basis of the result of the first analysis process, that the analyte identical to the analyte stored in the urine reservoir 70 is to be subjected to the second analysis process. The analyte in the urine reservoir 70 is supplied to the second analysis unit A2 through the urine flow channel 41c.

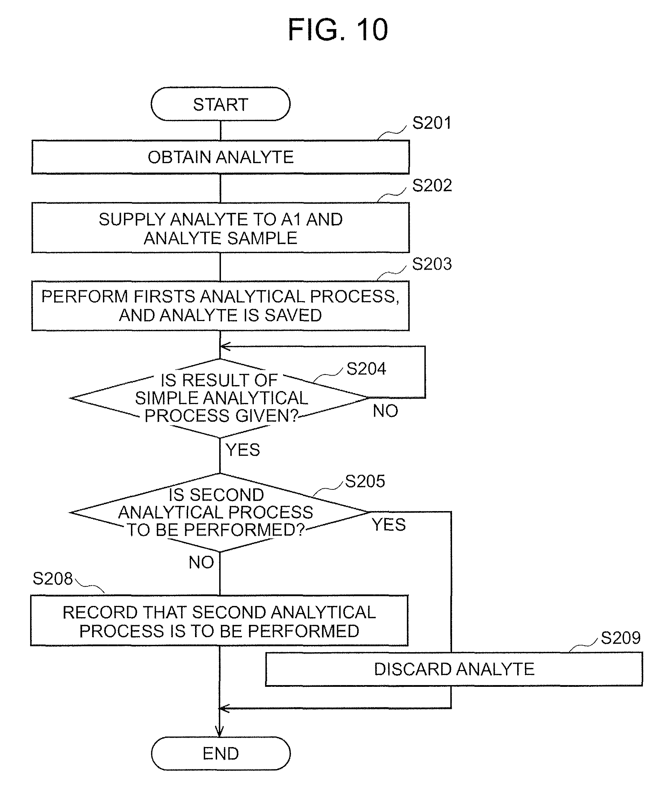

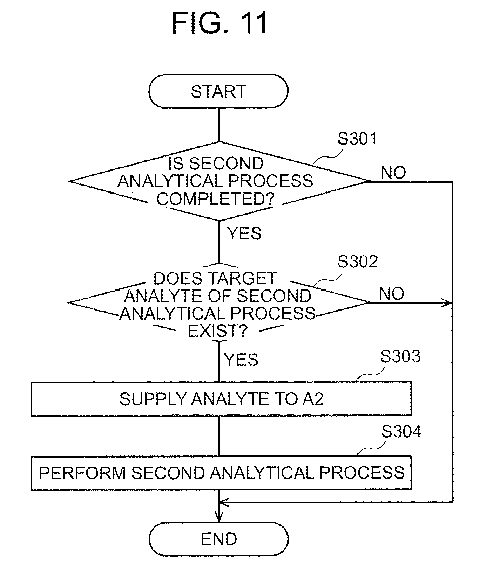

Flow of the analysis process of the urine analysis device according to this embodiment will be described with reference to the flowchart shown in FIGS. 10 and 11. Herein, the flow of the analysis process shown in FIG. 10 and the flow of the analysis process shown in FIG. 11 are performed independently, Note that each flow is actualized by the program stored in the memory 60B of the control unit 60.

In the flow of the analysis process shown in FIG. 10, to begin with, an analyte to be analyzed is obtained from the spitz 30 by the nozzle 4 at step S201. Next, the obtained analyte is supplied to the simple analysis unit A3 and the first analysis unit A1 at step S202. Then, at step S103, the first analysis process to the supplied analyte is performed by the simple analysis part A3. Further, the analyte supplied to the urine reservoir 70 is stored within the urine reservoir 70. On this occasion, the analyte is stored in one of the urine reservoirs 70, which dose not store the analyte. Note that, in this embodiment, this step S203 corresponds to the first analysis step and the sample storing step of the analysis method according to the second invention.

Then, at step S204, it is judged whether or not the result of the first analysis process by the simple analysis unit A3 is obtained. When the result of the first analysis process is obtained, at step 205, it is judged, on the basis of the result of the simple analysis process, whether or not the second analysis process to the same analyte is to be performed. In the present embodiment, the judging part 65 of the control unit 60 performs this step S205 of judging whether or not the second analysis process is to be performed. Note that, in the present embodiment, this step S205 corresponds to the judging step of the analysis method according to the second invention.

When it is judged, on the basis of the result of the first analysis process, that the second analysis process is to be performed, the judgment result is stored in the control unit 60 in a way that associates the judgment result with the analyte stored in the urine reservoir 70. In this case, the analyte stored in the urine reservoir 70 is leaved intact. On the other hand, when it is judged, on the basis of the result of the simple analysis process, that the second analysis process is not to be performed, the analyte stored in the urine reservoir 70 is discarded by a discarding device at step S207. Note that the discarding device is not illustrated in FIG. 8. The urine reservoir 70, the analyte in witch is discarded, is cleaned with cleaner fluid.

In flow of the analysis process, when the first analysis process to an analyte is completed, the next analysis target analyte is immediately obtained from the spitz 30 by the nozzle 4. That is, steps S201-S207 are re-performed immediately.

In the flow of the analysis process shown in FIG. 11, to begin with, it is judged at step S301 whether or not the second analysis process to the analyte, which is the last analysis target of the second analysis process, is completed. When it is judged that the second analysis process is completed, it is then judged at step S302 whether or not the analyte, the second analysis process to which is to be performed, is stored in any one of the reservoirs 70. This judgment is performed on the basis of the judgment result on necessity of the second analysis process stored in the control unit 60.

When it is judged that the analyte, the second analysis process to which is to be performed, is stored in a reservoir 70, the analyte in the reservoir 70 is supplied to the analysis unit A2 at step S303. Then, the second analysis process to the supplied analyte is performed at step S304.

According to the above-mentioned flow of the analysis process, a portion of the analyte obtained by the nozzle 4, which is supplied toward the urine reservoir 70, is stored in the urine reservoir 70. When the second analysis process is performed in the second analysis unit A2, the analyte is supplied from the urine reservoir 70 to the second analysis unit A2. It is therefore possible to advance the first analysis process in the first analysis unit A1, independent of progress state of the second analysis process in the second analysis unit A1. In other words, it is possible to start the first analysis process to the next analysis target analyte immediately after the first analysis process to the current analysis target analyte is completed. It is therefore possible to improve overall performance of the analysis process.

The nozzle 4 is the only nozzle arranged in the urine analysis device A according to the present embodiment to obtain the analyte. Consequently, in this embodiment, the first analysis process to an analyte and the second analysis process to the analyte are performed at different timings. When moving the rack 3 so as to get another analyte to be analyzed according to the progress state of the first analysis process, the spitz 30 holding an analyte is moved between a execution timing of the first analysis process to the analyte and a execution timing of the second analysis process to the analyte.

Therefore, to get the analysis target analyte at appropriate timings for the first and second analysis processes, it is necessary to match the movement of the nozzle 4 with the movement of the spitz 30.

However, in this embodiment, the analyte, which is supplied to the second analysis unit A2 when the second analysis process is judged to be performed, can be stored in the urine reservoir 70. It is therefore not necessary to match the movement of the nozzle 4 with the movement of the spitz 30. In other words, use of the urine reservoir 70 facilitates sharing of the nozzle 4 between the first analysis unit A1 and the second analysis unit A2. Further, as stated above, sharing the nozzle 4 between the first analysis unit A1 and the second analysis unit A2 make it possible to improve overall performance of the analysis process.

Note that, in the analysis device A according to the present invention, as in the analysis device A according to the first embodiment, it is not necessary to judge whether or the second analysis process to be performed to each and every analyte. For instance, to the analyte that is designated to perform the second analysis process thereto regardless of the result of the first analysis process, the first analysis process and second analysis process may be performed simultaneously. In this case, it is not necessary to store the analyte in the urine reservoir. Moreover, it is not necessary to store the reservoir 70 with the analyte that is designated not to perform the second analysis process regardless of the result of the first analysis process.

Computer program to realize the analysis process flow shown in FIGS 10 and 11 can be stored in a recording media readable by a computer (control unit 60). Then, the flow can be realized by making a computer read and execute the program.

Herein, the computer readable recording medium connotes a recording medium capable of storing information such as data and programs electrically, magnetically, optically, mechanically or by chemical action, which can be read from the computer. Among these recording mediums, those demountable from the computer are, for example, a floppy (registered trademark) disk, a magneto-optic disc, a CD-ROM, a CD-R/W, a DVD, a DAT, an 8mm tape, a memory card, etc. Further, a hard disk, a ROM (Read-Only Memory) and the like are given as the recording mediums fixed within the computer.

### (MODIFIED EXAMPLES)

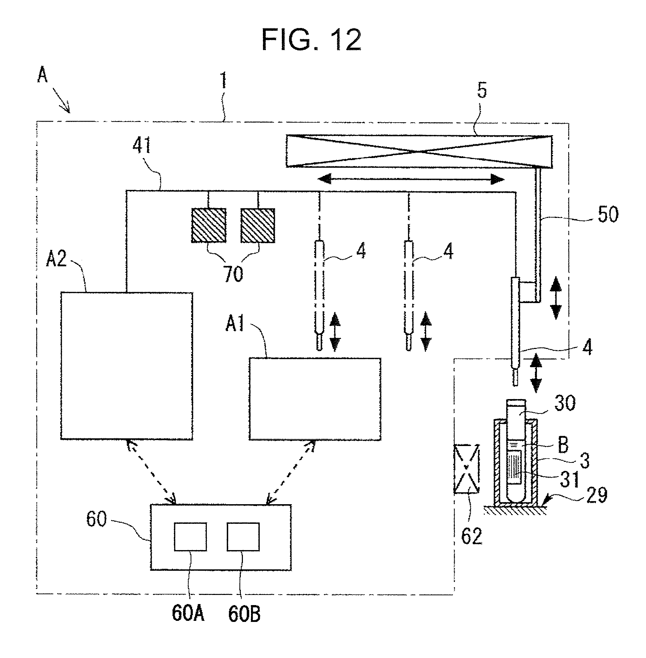

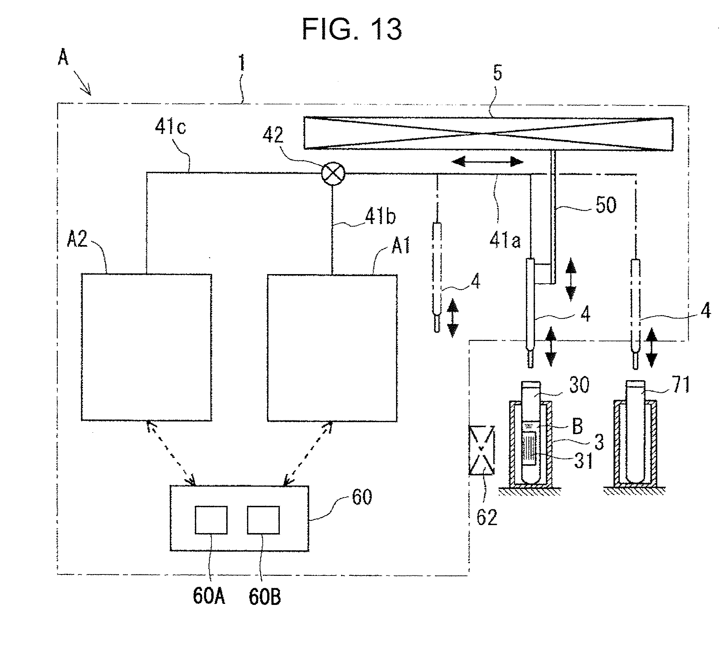

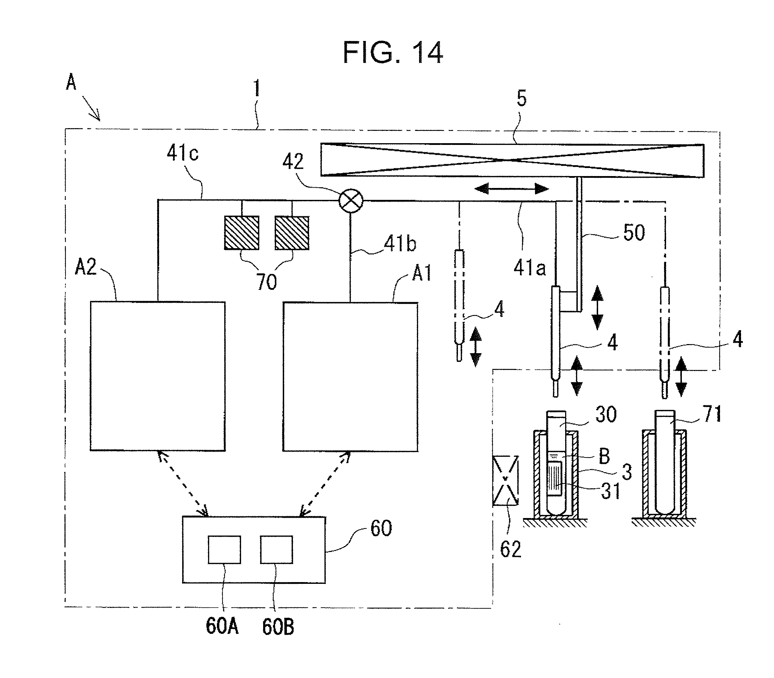

Outline of configuration of urine analysis devices according to modified examples of the aforementioned second embodiment will be described referring to FIGS. 12 through 14. FIG. 12 is a block diagram illustrating configuration of an urine analysis device according to a first modified example. FIG. 13 is a block diagram illustrating configuration of an urine analysis device according to a second modified example. FIG. 14 is a block diagram illustrating configuration of an urine analysis device according to a third modified example. Note that the appearance of the urine analysis unit according to a fourth modified example is the same with the appearance of the urine analysis device according to the third modified example of the first embodiment shown in Fig. 7.

As shown in FIG. 12, the urine analysis device A according to the first modified example includes, as with the second modified example of the first embodiment, the first analysis unit A1 that dispenses a drop of urine to be analyzed on a test strip, and performs urine qualitative test using the test strip. The nozzle moving device 5 and the urine flow channel 41 of this modified example are the same with the second modified example of the first embodiment. The urine reservoir 70 is fixed to the urine flow channel 41.

With this configuration, the analyte that is obtained by the nozzle 4 can be stored in the urine reservoir 70, and the analyte, which is to be subjected to the second analysis process, can be supplied from the urine reservoir 70 to the second analysis unit A2.

As shown in FIG. 13, the urine analysis device A according to the second modified example does not have the urine reservoirs 70 provided in its inside. The urine analysis device A according to this modified example includes, instead of the urine reservoirs 70, a plurality of spitzes 71, each of which serves as the urine reservoir, arranged on the outside of the device. The spitzes 71 are in the condition where they are held upright in a rack. The spitz 71 is the spitz that differs from the spitz 30 that is carried on the route shown in FIG. 1. That is, the spitz 71 is empty before being supplied with analyte from the nozzle 4. Note that the number of the spitzes 71 is not necessarily need to be larger than one.

In this modified example, the nozzle 4 can move to the position where the spitz 71 is set.
That is, the analyte sucked from the spitz 30 by the nozzle 4 can be discharged to the spitz 71. Further, the analyte stored in the spitz can be re-sucked by the nozzle 4. Consequently, the spitz 71 can work as the urine reservoir 70. In short, in this modified example, the require amount of analyte for the second analysis process is obtained from the spitz 30 by the nozzle 4, and the analyte is discharged to the spitz 71 by the nozzle 4 in order to store it in the spitz 71. Then, when the second analysis process to the analyte is performed, the analyte is sucked by the nozzle 4 and the sucked analyte is supplied to the second analysis unit A2.

Thus, by arranging the spitz that serves as the urine reservoir outside of the urine analysis device A, it becomes possible to suppress the increasing in size caused by providing the urine reservoir inside of the device. Note that, in this modified example, the spitz corresponds to the sample reservoir or the external reservoir of the second invention. However, in this modified example, the urine reservoir that is arranged outside of the urine analysis device A is not limited to the spitz. For instance, as the urine reservoir, a harn cup can be arranged outside of the urine analysis device A.

As shown in FIG. 14, the urine analysis device A according to the third modified example has a plurality of urine reservoirs 70 arranged in its inside. Moreover, the urine analysis device A has a plurality of spitzes 71, which serves as external reservoirs, arranged on its outside. The spitzes 30 are in the condition where they are held upright in the analyte rack 3. With this configuration, the urine analysis device A can have more urine reservoirs. Therefore, the device can hold more analytes to be subjected to the second analysis process. As a result, it is possible to perform the first analysis process to more analytes sequentially. Consequently, it becomes possible to further improve overall performance of the analysis process.

The urine analysis device A according to the fourth example has, as with the urine analysis device A according to the third example of the first embodiment, the nozzle 4A to obtain from the spitz 30 the analyte for the first analysis unit A1 and the nozzle 4B to obtain from the spitz 30 the analyte for the second analysis unit A2. If the urine reservoir is arranged inside of the urine analysis device A, the urine reservoir is arranged on an intermediate part of the urine flow channel to flow the analyse from the nozzle 4 to the second analysis unit A2. If the urine reservoir (spitz, etc.) is arranged outside of the urine analysis device A, the urine reservoir is arranged near the nozzle 4B.

In the urine analysis device A according to this modified example, the spitz holding the analysis target analyte is carried from the side where the nozzle 4A exists to the side where the nozzle 4B exists. By providing the urine reservoirs in the urine analysis device A, it becomes unnecessary to restrict time or transportation distance for a spitz to move from the position under the nozzle 4A to the position under the nozzle 4B on the basis of the required time for the first or second analysis process. Accordingly, also with the configuration of this modified example, it is possible to improve overall performance of the analysis process and to miniaturize the urine analysis device A.

### <THIRD EMBODIMENT>

The present embodiment is the embodiment of each of the first invention and the second invention. Note that parts that correspond to the conventional technology in this embodiment are the same with those of the analysis device according to the first embodiment. Therefore, the following discussion will be focused on a different parts from those of the urine analysis device according to the first embodiment, and explanations of parts same with the device will be omitted.

### (CONFIGURATION OUTLINE)

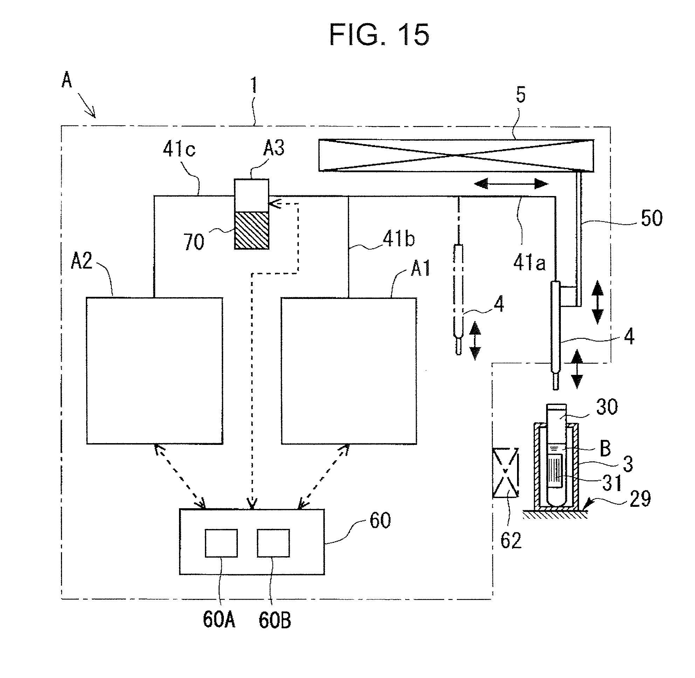

FIG. 15 is a block diagram illustrating the urine analysis device according to this embodiment. Note that the appearance of the urine analysis unit according to this embodiment is the same with the appearance of the urine analysis device according to the first embodiment shown in Fig. 1. In Fig. 15, the same numerals are employed as those used for the description of the urine analysis device according to the first embodiment in FIG. 5.

The urine analysis device A includes the simple analysis unit A3 similar to that of the first embodiment and the urine reservoir 70 similar to that of the first embodiment. However, in this embodiment, the simple analysis unit A3 is united with the urine reservoir 70, and the unit including the simple analysis unit A3 and the urine reservoir 70 are attached to an intermediate part of the second urine flow channel 41c.

With this configuration, the simple analysis process by the simple analysis unit A3 can be performed to the analyte stored in the urine reservoir 70. Consequently, both of the effect achieved by the urine analysis device according to the first embodiment and the effect achieved by the urine analysis device according to the first embodiment can be achieved.

### <FOURTH EMBODIMENT>

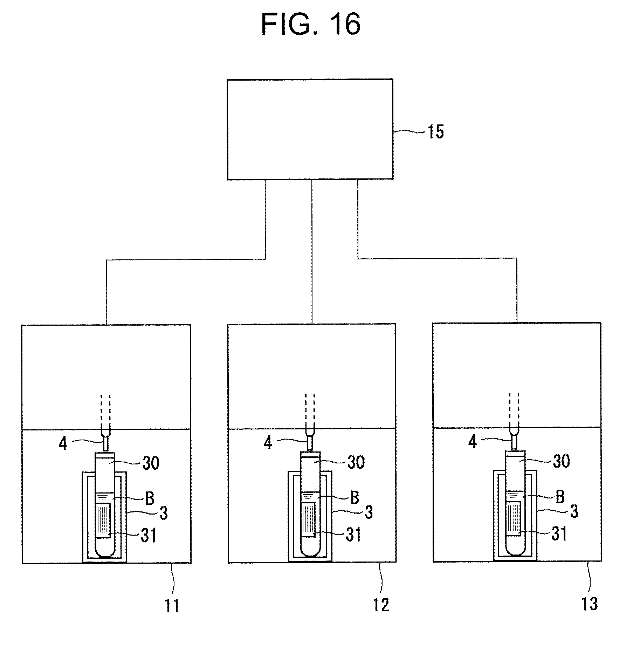

FIG. 16 illustrates configuration of an analysis system according to the fourth embodiment of the present invention.

The analysis system according to this embodiment is the modified version of the analysis device A according to the first embodiment. The configuration and operation of the analysis system according to the present embodiment will be explained in a way that puts a focus on differences from the urine analysis device A according to the first embodiment.

As shown in Figure, the analysis system includes a first analysis device 11, a second analysis device 12, a simple analysis device 13 and a management device 14. This analysis system is developed on the assumption that analysis devices 11-13 are installed on the places (e.g. different rooms) that are distant from each other. Note that, the analysis system is now described giving an example of the case where the analysis system is used in a way that three sample racks 3 of the same contents are prepared and are set to respective analysis units 11-13 almost at the same time. However, the present invention connotes the analysis system where the sample racks 3 are properly carried to respective analysis units 11-13 by a carry device (conveyance line).

The first analysis device 11 is a device that performs the first analysis process (urine qualitative test), which is similar to that of the first analysis unit A1, to analyte (urine in this embodiment) in each spitz 30 held by the analyte rack 3. The first analysis device 11 has a function of reading the identification code 31 attached to each spitz 30, a function of sending the read identification code 31 together with the analysis result of the analyte (concentrations of protein, glucose, hemoglobin, bilirubin and the like in the analyte, specific gravity of the analyte, etc.) and so on.

The simple analysis device 13 is a device that performs the simple analysis process, which is similar to that of the simple analysis unit A3, to analyte in each spitz 30 held by the analyte rack 3. This simple analysis device 13 has a function of reading the identification code 31 attached to each spitz 30, a function of sending the read identification code 31 together with the analysis result of the analyte (refractive index, specific gravity, value representing color, etc. of the analyte) and so on.

The second analysis device 12 is a device that performs the second analysis process, which is similar to that of the second analysis unit A2, to analyte in each spitz 30 held by the analyte rack 3. The second analysis device 12 also has a function of reading the identification code 31 attached to each spitz 30. Moreover, the second analysis device 12 has a function (described in detail later) of judging on the basis of the information from the management device 15 whether or not to perform analysis to the analyte in the spitz 30 to which an identification code 31.

The management device is a computer that collects the analysis results of the analyte by the analysis devices 11-13, and stores them into a prescribed database. This management device 15 has a function of determining which analyte is subjected to the analysis of the second analysis device 12.

Specifically, as already explained, the simple analysis device 13 is so configured as to send the read identification code 31 together with the analysis result of the analyte. When receiving information (information containing the identification code 31 and the analysis result) from the simple analysis device 13, the management device stores the information into the database, and judges whether or not the analyte (denoted hereinafter as the focused analyte) identified by the identification code 31 in the received information is to be subjected to the second analysis process. When judging that the focused analyte is to be subjected to the second analysis process, the management device 15 sends to the second analysis device 12 indication information which includes the identification code 31 of the focused analyte and indicates that the second analysis process is to the focused analyte to be performed. Whereas when judging that the focused analyte is not to be subjected to the second analysis process, the management device 15 sends to the second analysis device 12 indication information which includes the identification code 31 of the focused analyte and indicates that the second analysis process to the focused analyte is not to be performed.

The second analysis unit 12 receiving the indication information stores the indication information in its inside. Further, the second analysis unit 12 reads, before actually obtaining the analyte in a spitz 30, the identification code 31 attached to the spitz 30. Thereafter, the second analysis unit 12 retrieves the indication information which contains the read identification code 31 out of the indication information stored in its inside.

Then, the second analysis unit 12 starts the second analysis processing to the analyte if the retrieved indication information indicates that second analysis process is to be performed, and starts processing to the next analyte if the retrieved indication information indicates that second analysis process is not to be performed. Note that, the second analysis unit 12, when the indication information containing the read identification code 31 cannot be retrieved, watches and waits for the indication information containing the read identification code 31 from the management device 15, and performs the above-mentioned operation when receiving the indication information.

As obvious from the discussion given above, the analysis system according to this embodiment has such architecture that the second analysis process to a sample can be started before the first analysis process to the sample is completed. Consequently, according to this analysis system, it is possible to improve efficiency of analysis processing including two or more analysis processes to a sample.

The above embodiments can be combined to the greatest possible degree as the need arises.

### Industrial Applicability

The present invention is available to analysis of samples with two or more analysis processes.

### Reference Signs List

- A: urine analysis device
- A1: first analysis unit
- A2: second analysis unit
- A3: simple analysis unit
- 1: housing
- 2: carry system
- 3: analyte rack
- 4, 4A, 4B: nozzle
- 11: first analysis device
- 12: second analysis device
- 13: simple analysis device
- 15: management device
- 30: spitz
- 41a: common urine flow channel
- 41b: first urine flow channel
- 41c: second urine flow channel
- 60: control unit
- 61: first judging part
- 62: second judging part
- 63: judging part
- 70: urine reservoir
- 71: spitz

## Claims

1. An analysis device, comprising:
a short-time analysis unit to perform a short-time analysis process to a sample, which is completed in a shorter time than a first analysis process to the sample; and
a judging unit to judge, on a basis of a result of the short-time analysis process, whether or not to perform a second analysis process different from the first short-time analysis process.

2. The analysis device according to claim 1, further comprising a second analysis unit to perform the second analysis process to the sample.

3. The analysis device according to claim 2, wherein the judging unit, when judging that the second analysis process to the sample is to be performed, makes the second analysis unit start the second analysis process to the sample without waiting for completion of the first analysis process.

4. The analysis device according to any one of claims 1 to 3, further comprising a first analysis unit to perform the first analysis process to the sample.

5. The analysis device according to any one of claims 1 to 4, wherein the short-time analysis process is a process of measuring a specific physical property of the sample.

6. An analysis device, comprising:
a short-time analysis unit to measure a specific physical property of a sample;
a judging unit to judge, on a basis of a measurement result of the specific physical property by the short-time analysis unit, whether or not to perform a sediment test to the sample; and
a sediment testing unit to perform the sediment test to the sample, the sediment test to which is judged to be performed by the judging unit.

7. An analysis device of performing predetermined analysis processing to a sample, the device comprising:
a first analysis unit, a second analysis unit and a short-time analysis unit to perform a first analysis process to the sample, a second analysis process to the sample and a short-time analysis process to the sample, by which an analysis result is given in a shorter time than by the first analysis process, respectively; and
a judging unit to judge, before a result of the first analysis process is given, whether or not to perform the second analysis process on a basis of a result of the short-time analysis process.

8. The analysis device according to claim 7, wherein the judging unit serves as a first judging unit, and
further comprising a second judging unit to judge whether or not to perform the second analysis process on a basis of the result of the first analysis process.

9. The analysis device according to claim 8, wherein the second analysis process is performed when, after the first judging unit judges that the second analysis process to the sample is not to be performed, the second judging unit judges that the second analysis process to the same sample is to be performed.

10. The analysis device according to claim 8 or 9, wherein at least part of analysis items measured in the short-time analysis process are identical with or relevant to part of analysis items measured in the first analysis process.

11. The analysis device according to claim 10, wherein the part of the analysis items measured in the first analysis process are measured in the short-time analysis process in a way different from that of the first analysis process.

12. The analysis device according to claim 8 or 9, wherein an analysis item measured in the short-time analysis process differs from analysis items measured in the first analysis process.

13. The analysis device according to any one of claims 7 to 12, wherein the short-time analysis unit is fixed on a nozzle for getting a sample from a sample container, a flow channel through which the sample to be delivered to the first analysis unit flows, or a flow channel through which the sample to be delivered to the second analysis unit flows.

14. The analysis device according to any one of claims 7 to 13, further comprising a sample reservoir capable of temporarily storing the sample the second analysis process to which has been performed and the second analysis process to which has not been performed,
wherein the sample is delivered from the sample reservoir to the second analysis unit when the second analysis process is performed.

15. The analysis device according to claim 1.4, wherein the sample reservoir is united with the short-time analysis unit.

16. An analysis device of performing predetermined analysis processing to a sample, the device comprising:
a first analysis unit and a second analysis unit to perform a first analysis process to the sample and a second analysis process to the sample, respectively; and
a sample reservoir capable of temporarily storing the sample to be delivered to the second analysis unit.

17. The analysis device according to claim 16, further comprising a nozzle to get from a sample container the sample to be delivered to the first analysis unit and the second analysis unit.

18. The analysis device according to claim 16 or 17, further comprising judging means for judging whether or not to perform the second analysis process on a basis of a result of the first analysis process.

19. The analysis device according to claim 17 or 18, wherein the sample reservoir includes an internal reservoir fixed on a flow channel through which the sample to be delivered to the second analysis unit flows.

20. The analysis device according to claim 17 or 18, further comprising:
a first flow channel through which the sample to be delivered to the first analysis unit flows;
a second flow channel through which the sample to be delivered to the second analysis unit flows; and
a switching valve to supply the sample gotten by the nozzle to either the first flow channel or the second analysis unit,
wherein the sample reservoir includes an internal reservoir fixed on the second flow channel.

21. The analysis device according to any one of claims 17 to 20, wherein the nozzle gets the sample by sucking the sample from the sample container,
the sample reservoir includes an external reservoir to store the sample discharged from the nozzle, the sample reservoir being arranged at a position where the nozzle is able to discharge the sucked sample therein, and
the sample is sucked from the external reservoir by the nozzle when the sample is delivered to the second analysis unit.

22. The analysis device according to any one of claims 7 to 21, wherein the first and second analysis units are placed in a housing.

23. An analysis system to perform a specific analysis process to a sample, the analysis system comprising:
a first analysis device, a second analysis device, and a short-time analysis device to perform a first analysis process to the sample, a second analysis process to the sample, and a short-time analysis process to the sample, by which an analysis result is given in a shorter time than by the first analysis process, respectively; and
a judging device to judge whether or not to perform the second analysis process on a basis of a result of the first analysis process before a result of the first analysis process is given.

24. An analysis system to perform a specific analysis process to a sample, the analysis system comprising:
a first analysis device and a second analysis device to perform a first analysis process to the sample and a second analysis process to the sample, respectively; and
a sample reservoir capable of temporarily storing the sample to be delivered to the second analysis device.

25. An analysis method of performing a specific analysis process to a sample, the analysis method comprising:
a first analysis step, a second analysis step, and a short-time analysis step of performing a first analysis process to the sample, a second analysis process to the sample, and a short-time analysis process, by which an analysis result is given in a shorter time than by the first analysis process, respectively; and
a judging step of judging whether or not to perform the second analysis process on a basis of a result of the first analysis process before a result of the first analysis process is given.

26. The analysis method according to claim 25, wherein the judging step serves as a first judging step,
further comprising a second judging step of judging whether or not to perform the second analysis process on a basis of the result of the first analysis process.

27. The analysis method according to claim 26, wherein the second analysis process is performed when, after the first judging step judges that the second analysis process to the sample is not to be performed, the second judging step judges that the second analysis process to the same sample is to be performed.

28. The analysis method according to claim 26 or 27, wherein at least part of analysis items measured in the short-time analysis process are identical with or relevant to part of analysis items measured in the first analysis process.

29. The analysis method according to claim 28, wherein the part of analysis items measured in the first analysis process are measured in the short-time analysis process in a way different from that of the first analysis process.

30. The analysis method according to claim 26 or 27, wherein an analysis item measured in the short-time analysis process differs from analysis items measured in the first analysis process.

31. The analysis method according to any one of claims 25 to 30, further comprising a sample storing step of storing a sample reservoir with the sample that has been subjected to the simple analysis process and is not subjected to the second analysis process,
wherein the second analysis process is performed by using the analyte stored in the sample reservoir in the second analysis step.

32. Ananalysis method of performing a specific analysis process to a sample, the method comprising:
a first analysis step and a second analysis step of performing a first analysis process and a second analysis process, respectively; and
a sample storing step of temporarily storing the sample in a sample reservoir before the second analysis process is performed in the second analysis step.

33. The analysis method according to claim 32, further comprising a judging step of judging, on a basis of a result of the first analysis process, whether or not to perform the second analysis process.

34. A program for a computer that controls an analysis device to perform a specific analysis process to a sample, the analysis device including a first analysis unit, a second analysis unit, and a short-time unit to perform a first analysis process to the sample, a second analysis process to the sample, and a short-time analysis process to the sample, which is completed in a shorter time than a first analysis process to the sample, respectively, the program makes the computer execute:
a judging step of judging whether or not to perform the second analysis process on a basis of a result of the first analysis process before a result of the first analysis process is given.

35. The program according to claim 34, wherein the judging step serves as a first judging step, and
the program makes the computer further execute a second judging step of judging whether or not to perform the second analysis process on a basis of a result of the first judging step.

36. The program according to claim 35, the program makes the computer execute a step of making the second analysis unit start the second analysis process when, after the first step judges that the second analysis process to the sample is not to be performed, the second step judges that the second analysis process to the same sample is to be performed.

37. A program for a computer that controls an analysis device to perform a specific analysis process to a sample, the analysis device including a first analysis unit and a second analysis unit to perform a first analysis process to the sample and a second analysis process to the sample, respectively, and a sample reservoir capable of storing the sample, the program makes the computer execute:
a sample storing step of temporarily storing the sample in the sample reservoir before the second analysis process is performed in the second analysis step.

38. The program according to claim 37, the program makes the computer further execute a judging step of judging whether or not to perform the second analysis process on a basis of a result of the first analysis process.

39. A recording medium stored with the program according to any one of claims 34 to 38.
